(19) Europäisches Patentamt

European Patent Office

Office européen des brevets

(11) **EP 0 736 511 B1**

(12) **EUROPÄISCHE PATENTSCHRIFT**

(45) Veröffentlichungstag und Bekanntmachung des Hinweises auf die Patenterteilung:
**27.10.1999  Patentblatt 1999/43**

(51) Int Cl.⁶: **C07C 68/00**, C07C 69/96

(21) Anmeldenummer: **96104710.7**

(22) Anmeldetag: **25.03.1996**

(54) **Verfahren zur Herstellung von Diarylcarbonaten**

Process for the preparation of diaryl carbonates

Procédé de préparation de carbonates de diaryl

(84) Benannte Vertragsstaaten:
**DE ES FR GB IT NL**

(30) Priorität: **05.04.1995  DE 19512616**

(43) Veröffentlichungstag der Anmeldung:
**09.10.1996  Patentblatt 1996/41**

(73) Patentinhaber: **BAYER AG**
**51368 Leverkusen (DE)**

(72) Erfinder:
• **Buysch, Hans-Josef, Dr.**
  **47809 Krefeld (DE)**
• **Hesse, Carsten, Dr.**
  **47800 Krefeld (DE)**
• **Rechner, Johann, Dr.**
  **47906 Kempen (DE)**

(56) Entgegenhaltungen:
**EP-A- 0 507 546**          **DE-A- 2 815 501**

**Beschreibung**

Die vorliegende Erfindung betrifft ein Verfahren zur Herstellung von Diarylcarbonaten durch Umsetzung einer aromatischen Hydroxyverbindung (z.B. Phenol) mit Kohlenmonoxid und Sauerstoff bei erhöhter Temperatur in Gegenwart einer Base, eines quartären Salzes, eines Katalysators und eines Cokatalysators, das dadurch gekennzeichnet ist, daß man in Gegenwart eines heterogenen Promotors arbeitet.

Es ist bekannt, organische Carbonate durch oxidative Umsetzung von aromatischen Hydroxyverbindungen mit Kohlenmonoxid in Gegenwart eines Platinmetall-Katalysators herzustellen (DE-OS 28 15 512). Als Platinmetall wird bevorzugt Palladium eingesetzt. Zusätzlich können ein Cokatalysator (z.B. Mangan- oder Kobaltsalze), eine Base, ein quartäres Salz, verschiedene Chinone bzw. Hydrochinone und Trockenmittel eingesetzt werden. Dabei kann in einem Lösungsmittel, bevorzugt in Methylenchlorid, gearbeitet werden.

Mit diesen Verfahren sind nur geringe Raum-Zeit-Ausbeuten erzielbar, so daß lange Verweilzeiten erforderlich sind. Während der Reaktion ist dabei eine rasch sinkende Aktivität des Katalysators zu beobachten. Schließlich kommt die Aktivität des Katalysators völlig zum Erliegen.

Die in der EP-503 581 vorgeschlagene Verwendung von verschiedenen Kupfersalzen als Cokatalysator führt nicht zu höheren Raum-Zeit-Ausbeuten. Auch hier sinkt die Aktivität des Katalysators stetig und schnell, bis ein völlig inaktiver Katalysator vorliegt.

Der in verschiedenen Anmeldungen (EP 350 697, EP 350 700, EP 450 442, EP 503 581, EP 550 743, etc.) vorgeschlagene Einsatz erheblicher Mengen verschiedener Chinone bzw. Hydrochinone als Elektronen-Transfer-Katalysator verbessert weder die Raum-Zeit-Ausbeute, noch wird das Problem der Desaktivierung des Katalysators gelöst. Im Gegenteil wird die Selektivität und damit die Wirtschaftlichkeit des Prozesses durch die Bildung von Nebenprodukten erheblich gesenkt.

Die Anmeldungen EP 583 935, EP 583 937, EP 583 938 beziehen sich auf einen Kobalt-Cokatalysator mit einem fünfzähnigen Liganden, speziell das Kobalt-di-(salicylyl)-3,3'-diamino-N-methyldipropylamin (CoSMDP), der in Verbindung mit Terpyridin eingesetzt wird. Die Synthese dieses Komplexes ist vielstufig und aufwendig. Unter den angegebenen Reaktionsbedingungen sind diese Verbindungen nicht stabil, so daß die Verluste durch Zersetzung kostenintensiv ersetzt werden müssen, was eine wirtschaftliche Durchführung dieses Verfahrens unmöglich macht. Selbst durch den Einsatz derartiger, hochaktiver und schwierig herzustellender Cokatalysatoren kann nicht verhindert werden, daß die Aktivität des Katalysators schon nach 2 Stunden auf die Hälfte zurückgeht.

Es bestand daher die Aufgabe, ein Katalysatorsystem mit hoher Aktivität und hoher Standzeit zu finden, das die Herstellung aromatischer Carbonate unter wirtschaftlichen und technisch realisierbaren Bedingungen erlaubt.

Überraschenderweise wurde nun gefunden, daß man die Standzeit des Katalysators bei der Herstellung von Diarylcarbonaten durch oxidative Umsetzung einer aromatischen Hydroxyverbindung in Gegenwart eines Platinmetall-Katalysators, eines Cokatalysators, eines quartären Salzes und einer Base verlängern kann, wenn man in Gegenwart eines heterogenen Promotors arbeitet. Der Grund für die Desaktivierung des Platinmetall-Katalysators ist nicht bekannt. Um so überraschender ist die Tatsache, daß durch Zusatz einer inerten Komponente, als die ein heterogener Promotor angesehen werden muß, die Standzeit des Platinmetall-Katalysators verlängert werden kann.

Die Erfindung betrifft demnach ein Verfahren zur Herstellung von Diarylcarbonaten der Formel

$$RO - CO - OR$$

durch Umsetzung einer aromatischen Hydroxyverbindung der Formel

$$ROH,$$

wobei in den Formeln

R substituiertes oder nichtsubstituiertes $C_6$-$C_{12}$-Aryl, bevorzugt substituiertes oder nichtsubstituiertes Phenyl, besonders bevorzugt nichtsubstituiertes Phenyl bedeutet,

mit CO und $O_2$ bei einer Temperatur von 30 bis 200°C, bevorzugt 30 bis 150°C, besonders bevorzugt 40 bis 120°C, und einem Druck von 1 bis 150 bar, bevorzugt 2 bis 50 bar, besonders bevorzugt 5 bis 25 bar, in Gegenwart eines quartären Salzes, einer Base, eines Platinmetall-Katalysators und eines Cokatalysators, dadurch gekennzeichnet, daß man zusätzlich in Gegenwart eines heterogenen Promotors arbeitet, der eine Verbindung der Formel

$$A_xB_yC_z$$

darstellt, in der

A und B    unabhängig voneinander ein Element der Gruppen IIIA, IVA, VA, IIIB, IVB, VB, VIB oder VIIB des Periodensystems der Elemente (Mendelejew) bedeuten,

C    für ein Element der 2. Periode des Periodensystems der Elemente (Mendelejew) steht,

x    eine Zahl von 1 bis 3,

y    eine Zahl von 0 bis 3 und

z    eine Zahl von 1 bis 12 bedeuten.

Am Beispiel der Bildung von Diphenylcarbonat kann das erfindungsgemäße Verfahren wie folgt formelmäßig dargestellt werden:

$$2\ C_6H_5\text{-}OH + CO + \tfrac{1}{2}\,O_2 \rightarrow (C_6H_5O)_2CO + H_2O.$$

Bei den in das erfindungsgemäße Verfahren einzusetzenden heterogenen Promotoren handelt es sich bevorzugt um Verbindungen, in denen C für Bor, Kohlenstoff, Stickstoff oder Sauerstoff steht.

Bevorzugt werden Metalloxide, -carbide, -nitride oder -boride oder ein kohlenstoffhaltiges Material eingesetzt.

Als Beispiele für solche Verbindungen seien, ohne das erfindungsgemäße Verfahren auf diese einzuschränken, α-Aluminiumoxid, γ-Aluminiumoxid, Aluminiumcarbid, Aluminiumnitrid, Siliciumdioxid, Siliciumcarbid, Siliciumnitrid, Titandioxid, Titanborid, Titancarbid, Titannitrid, Zirkoniumdioxid, Zirkoniumborid, Zirkoniumnitrid, Cerdioxid, Dysprosiumoxid, Vanadiumpentoxid, Vanadiumborid, Vanadiumcarbid, Vanadiumnitrid, Molybdäncarbid, Wolframborid, Wolframnitrid, Wolframcarbid, Mangandioxid, Bismutoxid, Bleimolybdat und Bleititanat genannt.

Weiterhin bevorzugt handelt es sich bei den im erfindungsgemäßen Verfahren einzusetzenden Promotoren um kohlenstoffhaltige Materialien, wobei A als Element der Gruppe IVA ebenso wie C Kohlenstoff bedeutet und y Null ist. Beispiele für kohlenstoffhaltige Materialien sind Ruß, Graphit, Kohlenstoffasern und poröse Kohlenstoffe, wie Koks, Holzkohle und Aktivkohle. Bevorzugt werden Ruß bzw. Aktivkohle verwendet. Für den Einsatz im erfindungsgemäßen Verfahren können Aktivkohlen und Ruße aus pflanzlichen (z.B. Holz, Torf, Nußschalen, Kaffeebohnen), tierischen (z. B. Blut, Knochen) oder mineralischen Rohstoffen (z.B. Braunkohle, Steinkohle, petrochemische Kohlenwasserstoffe) verwendet werden.

Geeignete Aluminiumoxide können in kristalliner Form in verschiedenen Modifikationen vorliegen, beispielsweise als α-Aluminiumoxide, γ-Aluminiumoxide, η-Aluminiumoxide, κ-Aluminiumoxide oder ρ-Aluminiumoxide. Sie können aber auch amorphe Anteile enthalten. Es können natürlich vorkommende oder synthetische Aluminiumoxide verwendet werden. Die Aluminiumoxide, vorzugsweise natürlich vorkommende, können in geringem Maß andere Elemente wie Alkali- und Erdalkalimetalle, Eisen oder Silizium enthalten. Bevorzugt werden Produkte mit Gehalten an solchen Verunreinigungen von <2 Gew.-%, besonders bevorzugt <1 Gew.-%, verwendet. Besonders rein sind synthetische Aluminiumoxide. Es können saure, neutrale und basische Oxide verwendet werden. Derartige Aluminiumoxide und deren Herkunft bzw. Herstellverfahren für solche Verbindungen sind beispielsweise in Kirk-Othmer, Encyclopedia of Chemical Technology, 3. Aufl., Bd. 2, S. 218 ff., New York 1978, oder Ullmann's Encyclopedia of Industrial Chemistry, 5. Aufl., Bd. Al, S. 557 ff., Weinheim 1985, beschrieben. Dabei kommen sowohl die Aluminiumoxide aus natürlichen Quellen, d.h. aus verschiedenen Aluminiummineralien, als auch solche aus anderen Aluminiumvorprodukten, wie Aluminiumsalzen, Aluminiumalkoxiden und Aluminiumorganylen, in Frage. Bevorzugte Aluminiumoxide im Sinne der Erfindung sind sogenannte "aktivierte Aluminiumoxide". Diese können amorph, teilkristallin oder kristallin (z.B. γ-Al$_2$O$_3$ oder η-Al$_2$O$_3$) sein. Bevorzugte Aluminiumoxide sind weiterhin auch α-Aluminiumoxide mit BET-Oberflächen ≥2 m²/g.

Geeignete Titanoxide können in der orthorhombischen (Brookit) oder tetragonalen Modifikation (Anatas, Rutil) eingesetzt werden, aber auch amorphe Anteile enthalten. Derartige Titanoxide und deren Herkunft bzw. Herstellverfahren für solche Verbindungen sind beispielsweise in Kirk-Othmer, Encyclopedia of Chemical Technology, 3. Aufl., Bd. 17, S. 801 ff., New York 1978, sowie Bd. 23, S. 139 ff. beschrieben. Für den Einsatz in das erfindungsgemäße Verfahren kommen sowohl Titanoxide aus natürlichen Quellen, d.h. aus verschiedenen Titanmineralien, als auch synthetische aus anderen Titanvorprodukten, wie Titansalzen, Titanhalogeniden, Titanalkoxiden und Titanorganylen, in Frage. Ebenfalls in das erfindungsgemäße Verfahren einzusetzende Titanoxide sind auch solche, deren Oberfläche nachträglich modifiziert wurde und die in dieser Form kommerziell erhältlich sind. Die Titanoxide können in geringem Maß andere Elemente, wie Alkali- und Erdalkalimetalle, Eisen oder Silizium, enthalten. Bevorzugt werden Produkte mit Gehalten an solchen Verunreinigungen von <2 Gew.-%, besonders bevorzugt <1 Gew.-%, verwendet. Besonders rein sind synthetische Titanoxide.

Zirkoniumoxide können in verschiedenen Modifikationen auftreten, die bei bestimmten Temperaturen und Drücken z.T. reversibel ineinander überführbar sind. Zirkoniumoxide und deren Herkunft bzw. Herstellverfahren für solche Verbindungen sind beispielsweise in Kirk-Othmer, Enzyclopedia of Chemical Technology, 2. Aufl., Bd. 11, S. 729 ff., New York 1978, sowie Bd. 24, S. 882 ff. oder Ullmann's Encyclopedia of Industrial Chemistry, 4. Aufl., Bd. 24, S. 695 ff., Weinheim 1983, beschrieben. Für den Einsatz im erfindungsgemäßen Verfahren kommen sowohl die Zirkoniumoxide aus natürlichen Quellen, d.h. aus verschiedenen Zirkoniummineralien aber auch solche aus anderen Zirkoniumvorprodukten, wie Zirkoniumsalzen, Zirkoniumalkoxiden und Zirkoniumorganylen, in Frage. Bevorzugte Zirkoniumoxide im Sinne der Erfindung sind sogenannte "stabilisierte Zirkoniumoxide", die geringe Mengen Calciumoxid, Magnesiumoxid oder Yttriumoxid enthalten können.

Geeignete Oxide der Lanthaniden können in verschiedenen Modifikationen auftreten, die bei bestimmten Temperaturen und Drücken z.T. reversibel ineinander überführbar sind. Unter den Oxiden der Lanthaniden sind Cerdioxid ($CeO_2$) und Dysprosiumoxid bevorzugt. Cerdioxid kann Abweichungen von der Stöchiometrie zeigen, so daß in der Formel $CeO_{2-x}$ x einen Wert von 0 bis 0,3 annehmen kann. Ceroxide und deren Herkunft bzw. Herstellverfahren für solche Verbindungen sind beispielsweise in Kirk-Othmer, Enzyclopedia of Chemical Technology, 3. Aufl., Bd. 11, S. 729 ff., New York 1978, sowie Bd. 24, S. 882 ff. oder Ullmann's Encyclopedia of Industrial Chemistry, 4. Aufl., Bd. 24, S. 695 ff., Weinheim 1983, beschrieben. Für den Einsatz in das erfindungsgemäße Verfahren kommen sowohl die Ceroxide aus natürlichen Quellen, d.h. aus verschiedenen Cermineralien als auch solche aus anderen Cervorprodukten, wie z.B. Ceroxalaten oder -hydroxiden in Frage. Es können natürlich vorkommende oder synthetische Ceroxide verwendet werden. Bevorzugte Ceroxide im Sinne der Erfindung können auch geringe Mengen anderer Lanthanide (z.B. in Form von $Pr_6O_{11}$) enthalten.

Geeignete Vanadiumoxide können in verschiedenen Modifikationen auftreten, aber auch amorphe Anteile enthalten. Bevorzugt ist Vanadiumpentoxid. Vanadiumoxide und deren Herkunft bzw. Herstellverfahren für solche Verbindungen sind beispielsweise in Kirk-Othmer, Enzyclopedia of Chemical Technology, 3. Aufl., Bd. 11, S. 729ff., New York 1978, sowie Bd. 24, S. 882ff. oder Ullmann's Encyclopedia of Industrial Chemistry, 4. Aufl., Bd. 24, S. 695 ff., Weinheim 1983, beschrieben. Für den Einsatz im erfindungsgemäßen Verfahren kommen sowohl die Vanadiumoxide aus natürlichen Quellen, d.h. aus verschiedenen Vanadiummineralien als auch solche aus anderen Vanadiumvorprodukten, wie Vanadiumsalzen, Vanadiumalkoxiden und Vanadiumorganylen, in Frage. Es können natürlich vorkommende oder synthetische Vanadiumoxide verwendet werden.

Bei für das erfindungsgemäße Verfahren geeigneten Nitriden handelt es sich um Verbindungen aus Stickstoff und einem Metall oder Halbmetall. Bevorzugt sind die sogenannten "metallartigen" Nitride, wie z.B. Vanadiumnitrid, Titannitrid oder Wolframnitrid, und die sogenannten "kovalenten" Nitride, wie z.B. Bornitrid, Siliziumnitrid oder Aluminiumnitrid. Nitride und Herstellverfahren für solche Verbindungen sind beispielsweise in Kirk-Othmer, Enzyclopedia of Chemical Technology, 3. Aufl., Bd. 15, S. 871 ff., New York 1978, oder Ullmann's Encyclopedia of Industrial Chemistry, 4. Aufl., Bd. 17, S. 315 ff., Weinheim 1979, beschrieben.

Bei für das erfindungsgemäße Verfahren geeigneten Carbiden handelt es sich um binäre Verbindungen von Elementen mit Kohlenstoff, die sich ganz allgemein durch Einwirkung von elementarem Kohlenstoff oder Kohlenwasserstoffen auf Metalle und Metallverbindungen bei entsprechend hohen Temperaturen herstellen lassen. Bevorzugt sind die sogenannten "metallartigen" Carbide, wie z.B. Chromcarbid, Hafniumcarbid, Molybdäncarbid, Niobcarbid, Tantalcarbid, Vanadiumcarbid, Titancarbid oder Wolframcarbid, und die sogenannten "kovalenten" Carbide, wie z.B. Borcarbid oder Siliciumcarbid. Carbide und Herstellverfahren für solche Verbindungen sind beispielsweise in Kirk-Othmer, Enzyclopedia of Chemical Technology, 3. Aufl., Bd. 4, S. 476 ff, New York 1978, oder Ullmann's Encyclopedia of Industrial Chemistry, 5. Aufl., Bd. A5, S. 61 ff., Weinheim 1986, beschrieben.

Bei für das erfindungsgemäße Verfahren geeigneten Boriden handelt es sich um Verbindungen von Metallen mit Bor, wie z.B. Aluminiumborid, Vanadiumborid, Titanborid, Zirkoniumborid, Eisenborid, Kobaltborid oder Wolframborid. Boride und Herstellverfahren für solche Verbindungen sind beispielsweise in Kirk-Othmer, Enzyclopedia of Chemical Technology, 3. Aufl., Bd. 4, S. 123 ff., New York 1978, oder Ullmann's Encyclopedia of Industrial Chemistry, 5. Aufl., Bd. A 4, S. 303 ff., Weinheim 1985, beschrieben.

Bevorzugte Promotoren besitzen BET-Oberflächen von 0,5 bis 1 500 $m^2/g$, besonders bevorzugte solche von 1 bis 1 500 $m^2/g$ und ganz besonders bevorzugte solche von 2 bis 1 400 $m^2/g$.

Die Promotoren können als Pulver oder Formkörper eingesetzt werden und nach der Umsetzung z.B. durch Filtration, Sedimentation oder Zentrifugieren abgetrennt werden. Für den Fall der Anordnung als Festbett werden die Promotoren vorzugsweise als Formkörper, z.B. als Kugeln, Zylinder, Stäbchen, Hohlzylinder, Ringe, usw. eingesetzt. Die heterogenen Promotoren werden beim Arbeiten als Suspension in Rührgefäßen oder Blasensäulen in Mengen von 0,001 bis 50 Gew.-%, bevorzugt von 0,01 bis 10 Gew.-%, besonders bevorzugt von 0,1 bis 5 Gew.-%, bezogen auf die Menge an aromatischer Hydroxyverbindung, eingesetzt. Im Falle einer kontinuierlichen Fahrweise im Gegen- oder Gleichstrom oder in der Rieselphase am Festbett-Promotor werden Belastungen von 0,1 bis 20 g aromatische Hydroxyverbindung pro g Promotor und Stunde, bevorzugt 0,2 bis 10 g, besonders bevorzugt 0,2 bis 5 g, eingestellt.

Die bei diskontinuierlichem Arbeiten verwendeten Promotoren können bei gleichen Einsatzstoffen ohne Reinigung wiederholt eingesetzt werden. Bei einem Wechsel der Einsatzstoffe werden die Promotoren zweckmäßig durch Extrahieren mit inerten Lösungsmitteln, wie sie beispielsweise weiter unten als Reaktionsmedien genannt sind, oder mit Alkoholen, wie Methanol, Ethanol, Isopropanol oder Butanol, mit Estern oder Amiden der Essigsäure oder durch Behandlung mit überhitztem Wasserdampf oder Luft gereinigt.

Bei kontinuierlicher Arbeitsweise können die eingesetzten heterogenen Promotoren über lange Zeit im Reaktor verbleiben. Eine Regenerierung kann gegebenenfalls durch Überleiten von überhitztem Wasserdampf, gegebenenfalls unter Zugabe von untergeordneten Mengen an Luft (etwa 0,1 bis 20 Gew.-%, bezogen auf die eingesetzte Wasserdampfmenge) bei 150 bis 800°C oder durch Überleiten von 0,01 bis 20 Gew.-% Sauerstoff enthaltenden Verdünnungsgasen, wie Stickstoff oder Kohlendioxid, oder durch Kohlendioxid allein bei 200 bis 800°C erfolgen. Die bevorzugte Regenerationstemperatur liegt bei 250 bis 700°C, besonders bevorzugt bei 250 bis 600°C.

Bei den in das erfindungsgemäße Verfahren einzusetzenden aromatischen Hydroxyverbindungen handelt es sich beispielsweise um Phenol, o-, m- oder p-Kresol, o-, m- oder p-Chlorphenol, o-, m- oder p-Ethylphenol, o-, m- oder p-Propylphenol, o-, m- oder p-Methoxyphenol, 2,6-Dimethylphenol, 2,4-Dimethylphenol, 3,4-Dimethylphenol, 1-Naphthol, 2-Naphthol und Bisphenol A, bevorzugt um Phenol. Allgemein handelt es sich im Falle einer Substitution der aromatischen Hydroxyverbindung um 1 oder 2 Substituenten in der Bedeutung von $C_1$-$C_4$-Alkyl, $C_1$-$C_4$-Alkoxy, Fluor, Chlor oder Brom.

Für das erfindungsgemäße Verfahren können beliebige sowohl organische als auch anorganische Basen oder Mischungen derselben verwendet werden. Als Beispiele für anorganische Basen seien, ohne das erfindungsgemäße Verfahren einzuschränken, Alkalimetallhydroxide und -carbonate, -carboxylate oder andere Salze schwacher Säuren sowie Alkalisalze von aromatischen Hydroxyverbindungen der Formel (II), z.B. Alkalimetallphenolate, genannt. Selbstverständlich können in das erfindungsgemäße Verfahren auch die Hydrate von Alkalimetallphenolaten eingesetzt werden. Als Beispiel für ein solches Hydrat sei hier, ohne das erfindungsgemäße Verfahren einzuschränken, Natriumphenolat-trihydrat genannt. Die Menge an zugesetztem Wasser ist jedoch vorzugsweise so bemessen, daß pro Mol Base maximal 5 Mol Wasser eingesetzt werden. Höhere Wasserkonzentrationen führen i.a. zu schlechteren Umsätzen und Zersetzung gebildeter Carbonate. Als organische Basen seien, ohne das erfindungsgemäße Verfahren einzuschränken, tertiäre Amine, die als organische Reste $C_6$- bis $C_{10}$-Aryl-, $C_7$- bis $C_{12}$-Aralkyl- und/oder $C_1$- bis $C_{20}$-Alkyl-Reste tragen können oder Pyridinbasen oder hydrierte Pyridinbasen darstellen, genannt, beispielsweise Triethylamin, Tripropylamin, Tributylamin, Trioctylamin, Benzyldimethylamin, Dioctylbenzylamin, Dimethylphenethylamin, 1-Dimethylamino-2-phenylpropan, Pyridin, N-Methylpiperidin, 1,2,2,6,6-Pentamethyl-piperidin. Bevorzugt wird als Base ein Alkalisalz einer aromatischen Hydroxyverbindung verwendet, besonders bevorzugt ein Alkalisalz der aromatischen Hydroxyverbindung, die auch zum organischen Carbonat umgesetzt werden soll. Diese Alkalisalze können Lithium-, Natrium-, Kalium-, Rubidium- oder Cäsiumsalze sein. Bevorzugt werden Lithium-, Natrium- und Kaliumphenolat, besonders bevorzugt Natriumphenolat, eingesetzt.

Die Base kann dem Reaktionsgemisch als reine Verbindung in fester Form oder als Schmelze zugesetzt werden. In einer weiteren Ausführungsform der Erfindung wird die Base dem Reaktionsgemisch als Lösung, die 0,1 bis 80 Gew.-%, bevorzugt 0,5 bis 65 Gew.-%, besonders bevorzugt 1 bis 50 Gew.-% der Base enthält, zugesetzt. Als Lösungsmittel können hierbei sowohl Alkohole oder Phenole, wie z.B. das umzusetzende Phenol, als auch inerte Lösungsmittel verwendet werden. Als solche seien die weiter unten als Reaktionsmedien erwähnten genannt. Diese Lösungsmittel können allein oder in beliebiger Kombination miteinander eingesetzt werden. So besteht eine Ausführungsform des erfindungsgemäßen Verfahrens beispielsweise darin, daß man die Base in einer Phenolschmelze löst, die mit einem Lösungsmittel verdünnt wurde. Bevorzugt wird die Base in der Schmelze einer aromatischen Hydroxyverbindung gelöst, besonders bevorzugt in einer Schmelze der aromatichen Hydroxyverbindung, die zum organischen Carbonat umgesetzt werden soll. Ganz besonders bevorzugt wird die Base, in Phenol gelöst, zugesetzt. Die Base wird in einer von der Stöchiometrie unabhängigen Menge zugesetzt. Das Verhältnis von Platinmetall zu Base wird vorzugsweise so gewählt, daß pro Mol Platinmetall 0,1 bis 500, bevorzugt 0,5 bis 200, besonders bevorzugt 0,9 bis 130, Äquivalente Base eingesetzt werden.

Das erfindungsgemäße Verfahren wird vorzugsweise ohne Lösungsmittel durchgeführt. Selbstverständlich können auch inerte Lösungsmittel verwendet werden. Als Beispiele für Lösungsmittel seien Dimethylacetamid, N-Methylpyrrolidinon, Dioxan, t-Butanol, Cumylalkohol, Isoamylalkohol, Tetramethylharnstoff, Diethylenglykol, halogenierte Kohlenwasserstoffe (z.B. Chlorbenzol oder Dichlorbenzol) und Ether genannt.

Die für das erfindungsgemäße Verfahren geeigneten Platinmetall-Katalysatoren bestehen aus mindestens einem Edelmetall der Gruppe VIIIB, vorzugsweise Palladium. Das Platinmetall kann bei dem erfindungsgemäßen Verfahren in verschiedener Form zugegeben werden. Palladium kann z.B. in metallischer Form oder bevorzugt in Form von Palladium-Verbindungen der Oxidationsstufen 0 bzw. +2, wie beispielsweise Palladium(II)-acetylacetonat, -halogenide oder einer Platinmetall-halogenid enthaltenden Komplexverbindung, die außerdem beispielsweise Olefine, Amine, Phosphine, Nitrile, Kohlenmonoxid oder Wasser enthalten kann, wie $A_2(PdHal_4)$, wobei A beispielsweise für Li, Na, K, $NH_4$, Rb, Cs, $NR_4$ mit R= organischer Rest $C_6$- bis $C_{10}$-Aryl-, $C_7$- bis $C_{12}$-Aralkyl- und/oder $C_1$- bis $C_{20}$-Alkyl-Rest, und Hal für ein Halogen, wie beispielsweise F, Cl, Br, I steht, -carboxylate von $C_2$-$C_6$-Carbonsäuren, -nitrat, -oxide oder andere Palladiumkomplexe, die beispielsweise Olefine, Amine, Phosphine, Nitrile, Kohlenmonoxid, Wasser und/oder Halogenide enthalten können, eingesetzt werden. Besonders bevorzugt sind Palladiumbromid und Palladiumacetylacetonat. Die Menge an Platinmetall-Katalysator ist im erfindungsgemäßen Verfahren nicht beschränkt. Bevorzugt wird so viel Katalysator zugesetzt, daß seine Konzentration, gerechnet als Metall, im Reaktionsansatz 1 bis 3 000 ppm beträgt, besonders bevorzugt sind Konzentrationen von 10 bis 1 000 ppm, ganz besonders bevorzugt von 20 bis 1 000 ppm.

Als Cokatalysator für das erfindungsgemäße Verfahren wird eine Metallverbindung der Gruppen IB, IIB, IIIB, IVB, VB, VIB oder VIIB des Periodensystems der Elemente (Mendelejew) eingesetzt, wobei das Metall in verschiedenen Oxidationsstufen eingesetzt werden kann. Ohne das erfindungsgemäße Verfahren einzuschränken, seien Mangan (II), Mangan(III), Kupfer(I), Kupfer(II), Kobalt(II), Kobalt(III), Vanadium(III) und Vanadium(IV) genannt. Die Metalle können beispielsweise als Halogenide, Oxide, Carboxylate von $C_2$-$C_6$-Carbonsäuren, Diketonate oder Nitrate sowie als Komplexverbindungen, die beispielsweise Kohlenmonoxid, Olefine, Amine, Phosphine und/oder Halogenide enthalten können, eingesetzt werden. Bevorzugt werden Manganverbindungen im erfindungsgemäßen Verfahren verwendet, besonders bevorzugt Mangan(II)komplexe, ganz besonders bevorzugt Mangan(II)acetylacetonat. Der Cokatalysator wird in einer solchen Menge zugesetzt, daß seine Konzentration im Bereich von 0,001 bis 20 Gew.-% des Reaktionsgemisches liegt, bevorzugt ist der Konzentrationsbereich von 0,005 bis 5 Gew.-%, besonders bevorzugt 0,01 bis 2 Gew.-%.

Bei den im Rahmen der vorliegenden Erfindung eingesetzten quartären Salzen kann es sich beispielsweise um mit organischen Resten substituierte Ammonium- oder Phosphoniumsalze handeln. Geeignet für den Einsatz im erfindungsgemäßen Verfahren sind Ammonium- und Phosphoniumsalze, die als organische Reste $C_6$-bis $C_{10}$-Aryl-, $C_7$- bis $C_{12}$-Aralkyl- und/oder $C_1$- bis $C_{20}$-Alkyl-Reste und als Anion ein Halogenid, Tetrafluoroborat oder Hexafluorophosphat tragen. Bevorzugt werden in das erfindungsgemäße Verfahren Ammoniumsalze, die genannten organischen Reste und als Anion ein Halogenid tragen, eingesetzt; besonders bevorzugt ist Tetrabutylammoniumbromid. Die Menge eines solchen quartären Salzes beträgt 0,1 bis 50 Gew.-%, bezogen auf das Gewicht des Reaktionsgemisches. Vorzugsweise beträgt diese Menge 0,5 bis 15 Gew.-%, besonders bevorzugt 1 bis 10 Gew.-%.

Das erfindungsgemäße Verfahren wird, vorzugsweise ohne Lösungsmittel, bei 30 bis 200°C, bevorzugt bei 30 bis 150°C, besonders bevorzugt bei 40 bis 120°C, und bei einem Druck von 1 bis 150 bar, bevorzugt von 2 bis 50 bar, besonders bevorzugt bei 5 bis 25 bar, durchgeführt.

Das erfindungsgemäße Verfahren kann in verschiedenen Ausführungsvarianten ausgeübt werden. Eine Möglichkeit besteht in der diskontinuierlichen Durchführung. Dabei werden CO und Sauerstoff entweder durch einen Begasungsrührer (im Falle eines Rührkessels) oder andere bekannte Gasverteilungsorgane in die Reaktionsmischung geleitet. Durch das überschüssige Reaktionsgas wird das Reaktionswasser kontinuierlich aus dem Reaktor entfernt. Nach Erreichen des vorgesehenen Umsatzes wird das Reaktionsgemisch aus dem Reaktor entfernt oder gegebenenfalls im Reaktor aufgearbeitet. In den bevorzugten Ausführungsformen des erfindungsgemäßen Verfahrens kommt eine kontinuierliche Arbeitsweise im Einzelreaktor oder in einer Kaskade aus Reaktoren zum Einsatz. Hierzu wird als Reaktor ein Rührkessel oder eine Blasensäule oder eine Kaskade aus solchen Reaktoren eingesetzt, wobei die Kaskade aus 2 bis 15, bevorzugt 2 bis 10, besonders bevorzugt 2 bis 5 Einzelreaktoren besteht.

Die folgenden Beispiele verdeutlichen das erfindungsgemäße Verfahren, ohne es jedoch auf diese einzuschränken.

## Beispiele 1-13

In einem Planschlifftopf mit Begasungsrührer, Kühler und nachgeschalteter Kühlfalle wurden 0,078 g Palladiumbromid und 5 g Tetrabutylammoniumbromid bei 55°C in 300 g Phenol gelöst. Durch diese Lösung wurde zur Aktivierung des Katalysators eine Stunde lang Kohlenmonoxid (20 l/h) geleitet. Dann wurden 0,76 g Mangan(II)acetylacetonat, 0,9 g Natriumphenolat und 3 g des heterogenen Promotors (siehe Tabelle) zugegeben und unter Einleitung eines Gasgemisches aus Kohlenmonoxid und Sauerstoff (95:5 Vol-%) gerührt. Die Menge an Gasgemisch wurde auf 60 Nl/h eingestellt. Dem Reaktionsgemisch wurde jede Stunde eine Probe entnommen und gaschromatographisch analysiert. Die Analysen ergaben die in der Tabelle aufgezeichneten Gehalte an Diphenylcarbonat.

| Bsp. | Promotor | DPC-Gehalt [%] | | |
|---|---|---|---|---|
| | | 2 h | 15 h | 30 h |
| 1 | $V_2O_5$ | 1,23 | 9,37 | 18,77 |
| 2 | $MoC_2$ | 0,97 | 7,94 | 14,11 |
| 3 | $ZrO_2$ | 0,96 | 7,34 | 14,34 |
| 4 | $Al_2O_3$ (Rhone-Poulenc, SPH 512) | 0,69 | 6,09 | 12,63 |
| 5 | $CeO_2$ | 0,61 | 5,74 | 8,75 |
| 6 | Aktivkohle (Strem, Nr. 06-100) | 1,02 | 7,31 | 15,86 |
| 7 | $TiO_2$ (Bayertitan PK 5585) | 1,00 | 7,71 | 14,92 |
| 8 | $Bi_2O_3$ | 1,21 | 9,44 | 17,91 |
| 9 | Aktivkohle (Chemviron, Typ CPG) | 0,90 | 6,77 | 13,07 |
| 10 | $ZrB_2$ | 0,87 | 6,98 | 13,06 |
| 11 | AlN | 0,77 | 5,55 | 11,99 |
| 12 | $Si_3N_4$ | 1,03 | 7,63 | 15,35 |
| 13 | $PbTiO_3$ | 0,66 | 5,28 | 9,67 |

**Vergleichsbeispiel 1**

Der Versuch wurde, wie in Beispiel 1 beschrieben, wiederholt, jedoch wurde ohne einen heterogenen Promotor gearbeitet. Die gaschromatographische Analyse der Proben ergab, daß nach 2 Stunden 0,55 % Diphenylcarbonat, nach 15 Stunden 5,5 % Diphenylcarbonat und nach 30 Stunden 7,4 % Diphenylcarbonat im Reaktionsgemisch enthalten waren.

**Vergleichsbeispiel 2**

Der Versuch wurde, wie in Beispiel 1 beschrieben, wiederholt, jedoch wurde kein Palladiumbromid zugesetzt. Die gaschromatographische Analyse der Proben ergab, daß nach zehn Stunden kein Diphenylcarbonat im Reaktionsgemisch enthalten war. Dieses Beispiel zeigt, daß der Promotor allein keine katalytische Aktivität hat.

**Beispiel 14**

In einem Autoklaven (1 l) mit Begasungsrührer, Kühler und nachgeschalteter Kühlfalle wurden 0,34 g Palladiumbromid und 8,31 g Tetrabutylammoniumbromid bei 80°C in 450 g Phenol gelöst. Durch diese Lösung wurde zur Aktivierung des Katalysators eine Stunde lang Kohlenmonoxid (3 l/h) geleitet. Dann wurden 0,77 g Mangan(II)acetylacetonat und 2,21 g Natriumphenolat, gelöst in 50 g Phenol, und 10 g $V_2O_5$ zugegeben und unter Einleitung eines Gasgemisches aus Kohlenmonoxid und Sauerstoff (95:5 Vol.-%) der Druck auf 10 bar eingestellt. Die Menge an Gasgemisch wurde auf 300 Nl/h eingestellt. Dem Reaktionsgemisch wurde jede Stunde eine Probe entnommen und gaschromatographisch analysiert. Die Analysen ergaben, daß nach einer Stunde 13,0 % Diphenylcarbonat, nach 2 Stunden 18,0 % Diphenylcarbonat und nach 3 Stunden 21,5 % Diphenylcarbonat im Reaktionsgemisch enthalten waren. In der Kühlfalle waren 19,6 g eines Phenol/Wasser-Gemisches kondensiert.

**Beispiel 15**

In einem Autoklaven (1 l) mit Begasungsrührer, Kühler und nachgeschalteter Kühlfalle wurden 0,080 g Palladiumbromid und 8,31 g Tetrabutylammoniumbromid bei 90°C in 450 g Phenol gelöst. Durch diese Lösung wurde zur Aktivierung des Katalysators eine Stunde lang Kohlenmonoxid (3 l/h) geleitet. Dann wurden 0,77 g Mangan(II)acetylacetonat und 2,21 g Natriumphenolat, gelöst in 50 g Phenol, und 10 g $TiO_2$ (Bayertitan PK 5585) zugegeben und unter Einleitung eines Gasgemisches aus Kohlenmonoxid und Sauerstoff (95:5 Vol-%) der Druck auf 11 bar eingestellt. Die Menge an Gasgemisch wurde auf 300 Nl/h eingestellt. Dem Reaktionsgemisch wurde jede Stunde eine Probe entnommen und gaschromatographisch analysiert. Die Analysen ergaben, daß nach einer Stunde 12,1 % Diphenylcarbonat, nach 2 Stunden 17,5 % Diphenylcarbonat und nach 3 Stunden 20,3 % Diphenylcarbonat im Reaktionsgemisch enthalten waren. In der Kühlfalle waren 16,6 g eines Phenol/Wasser-Gemisches kondensiert.

**Beispiel 16**

In einer Blasensäule (1 l Volumen) mit einem Ring aus Begasungsdüsen, aufgesetztem Kühler und nachgeschalteter Kühlfalle wurden 0,080 g Palladiumbromid und 8,31 g Tetrabutylammoniumbromid bei 75°C in 450 g Phenol gelöst. Durch diese Lösung wurde zur Aktivierung des Katalysators eine Stunde lang Kohlenmonoxid (10 l/h) geleitet. Dann wurden 0,77 g Mangan(II)acetylacetonat und 2,21 g Natriumphenolat, gelöst in 50 g Phenol, und 1 g Aktivkohle (Strem, Nr. 06-100) zugegeben und unter Einleitung eines Gasgemisches aus Kohlenmonoxid und Sauerstoff (96,5: 3,5 Vol-%) der Druck auf 8 bar eingestellt. Die Menge an Gasgemisch wurde auf 300 Nl/h eingestellt. Dem Reaktionsgemisch wurde jede Stunde eine Probe entnommen und gaschromatographisch analysiert. Die Analysen ergaben, daß nach einer Stunde 9,3 % Diphenylcarbonat, nach 2 Stunden 15,2 % Diphenylcarbonat und nach 3 Stunden 18,3 % Diphenylcarbonat im Reaktionsgemisch enthalten waren. In der Kühlfalle waren 15,6 g eines Phenol-Wasser-Gemisches kondensiert.

**Patentansprüche**

1.  Verfahren zur Herstellung von Diarylcarbonaten der Formel

$$RO - CO - OR$$

durch Umsetzung einer aromatischen Hydroxyverbindung der Formel

$$ROH,$$

wobei in den Formeln

R    substituiertes oder nichtsubstituiertes $C_6$-$C_{12}$-Aryl, bevorzugt substituiertes oder nichtsubstituiertes Phenyl, besonders bevorzugt nichtsubstituiertes Phenyl bedeutet,

mit CO und $O_2$ bei einer Temperatur von 30 bis 200°C, bevorzugt 30 bis 150°C, besonders bevorzugt 40 bis 120°C, und einem Druck von 1 bis 150 bar, bevorzugt 2 bis 50 bar, besonders bevorzugt 5 bis 25 bar, in Gegenwart eines quartären Salzes, einer Base, eines Platinmetall-Katalysators und eines Cokatalysators, dadurch gekennzeichnet, daß man zusätzlich in Gegenwart eines heterogenen Promotors arbeitet, der eine Verbindung der Formel

$$A_xB_yC_z$$

darstellt, in der

A und B    unabhängig voneinander ein Element der Gruppen IIIA, IVA, VA, IIIB, IVB, VB, VIB oder VIIB des Periodensystems der Elemente (Mendelejew) bedeuten,

C        für ein Element der 2. Periode des Periodensystems der Elemente (Mendelejew) steht,

x        eine Zahl von 1 bis 3,

y        eine Zahl von 0 bis 3 und

z        eine Zahl von 1 bis 12 bedeuten.

2.  Verfahren nach Anspruch 1, dadurch gekennzeichnet, daß man den heterogenen Promotor in einer Menge von 0,001 bis 50 Gew.-%, bevorzugt 0,01 bis 10 Gew.-%, besonders bevorzugt 0,1 bis 5 Gew.-%, bezogen auf die Menge an aromatischer Hydroxyverbindung, einsetzt, wenn der Promotor als Suspension angewandt wird, oder eine Belastung von 0,1 bis 20 g, bevorzugt 0,2 bis 10 g, besonders bevorzugt 0,2 bis 5 g, an aromatischer Hydroxyverbindung pro g Promotor und Stunde einstellt, wenn der Promotor im Festbett angeordnet ist.

3.  Verfahren nach Anspruch 1, dadurch gekenzeichnet, daß in der Formel $A_xB_yC_z$ für den Promotor C ein Element aus der Gruppe von Bor, Kohlenstoff, Stickstoff und Sauerstoff darstellt.

4.  Verfahren nach Anspruch 3, dadurch gekennzeichnet, daß der Promotor ein Metalloxid, -carbid, -nitrid oder -borid oder ein kohlenstoffhaltiges Material darstellt.

5.  Verfahren nach Anspruch 1, dadurch gekennzeichnet, daß zu seiner Durchführung als Reaktor ein Rührkessel oder eine Blasensäule oder eine Kaskade aus solchen Reaktoren eingesetzt wird, wobei die Kaskade aus 2 bis 15, bevorzugt 2 bis 10, besonders bevorzugt 2 bis 5 Einzelreaktoren besteht.

6.  Verfahren nach Anspruch 1, dadurch gekennzeichnet, daß das Platinmetall Palladium ist, bevorzugt in Form von Palladiumverbindungen, besonders bevorzugt in Form von Palladiumbromid oder Palladiumacetylacetonat.

7.  Verfahren nach Anspruch 1, dadurch gekennzeichnet, daß man als organische Hydroxyverbindung Phenol einsetzt.

8.  Verfahren nach Anspruch 1, dadurch gekennzeichnet, daß man als Base ein tertiäres Amin, Alkaliphenolat oder Alkalisalz schwacher Säuren, vorzugsweise ein Alkalicarboxylat und/oder -phenolat, besonders bevorzugt Natriumphenolat verwendet.

9.  Verfahren nach Anspruch 1, dadurch gekennzeichnet, daß man als quartäres Salz ein Tetraalkylammonium- oder -phosphoniumsalz, bevorzugt ein Tetraalkylammoniumsalz, besonders bevorzugt Tetrabutylammoniumbromid einsetzt.

10. Verfahren nach Anspruch 1, dadurch gekennzeichnet, daß man aus Phenol, CO und Sauerstoff in Gegenwart von Natriumphenolat, Tetrabutylammoniumbromid und eines Katalysators Diphenylcarbonat herstellt.

**Claims**

1.  Process for the preparation of diaryl carbonates corresponding to the formula

$$RO - CO - OR$$

by reacting an aromatic hydroxyl compound corresponding to the formula

$$ROH,$$

wherein in the formulae

R   denotes substituted or unsubstituted $C_6$-$C_{12}$ aryl, preferably substituted or unsubstituted phenyl, particularly preferably unsubstituted phenyl,

with CO and $O_2$ at a temperature of 30°C to 200°C, preferably 30°C to 150°C, particularly preferably 40°C to 120°C, and at a pressure of 1 to 150 bar, preferably 2 to 50 bar, particularly preferably 5 to 25 bar, in the presence of a quaternary salt, of a base, of a platinum metal catalyst and of a cocatalyst, characterised in that the procedure is carried out additionally in the presence of a heterogeneous promoter, which is a compound corresponding to the formula

$$A_xB_yC_z,$$

wherein

## EP 0 736 511 B1

A and B, independently of one another, denote an element of the groups IIIA, IVA, VA, IIIB, IVB, VB, VIB or VIIB of the periodic system of elements (Mendeleev),

C represents an element of the second period of the periodic system of elements (Mendeleev),

x denotes a number from 1 to 3,

y denotes a number from 0 to 3 and

z denotes a number from 1 to 12.

2.  Process according to claim 1, characterised in that the heterogeneous promoter is used in a quantity of from 0.001 to 50 wt.%, preferably 0.01 to 10 wt.%, particularly preferably 0.1 to 5 wt.%, based on the quantity of aromatic hydroxyl compound, if the promoter is used in the form of a suspension, or at a loading of 0.1 to 20 g, preferably 0.2 to 10 g, particularly 0.2 to 5 g, of aromatic hydroxyl compound per g promoter and per hour, if the promoter is provided in a fixed bed.

3.  Process according to claim 1, characterised in that in the formula $A_xB_yC_z$ of the promoter, C is an element from the group including boron, carbon, nitrogen and oxygen.

4.  Process according to claim 3, characterised in that the promoter is a metal oxide, metal carbide, metal nitride or metal boride or a material containing carbon.

5.  Process according to claim 1, characterised in that the reactor used in order to carry it out is a stirred-tank reactor or a bubble column or a series of such reactors, the series comprising 2 to 15, preferably 2 to 10, particularly preferably 2 to 5 individual reactors.

6.  Process according to claim 1, characterised in that the platinum metal is palladium, preferably in the form of palladium compounds, particularly preferably in the form of palladium bromide or palladium acetylacetonate.

7.  Process according to claim 1, characterised in that the organic hydroxyl compound used is phenol.

8.  Process according to claim 1, characterised in that the base used is a tertiary amine, alkali metal phenolate or alkali metal salt of weak acids, preferably an alkali metal carboxylate and/or phenolate, particularly preferably sodium phenolate.

9.  Process according to claim 1, characterised in that the quaternary salt used is a tetraalkylammonium salt or tetraalkylphosphonium salt, preferably a tetraalkylammonium salt, particularly preferably tetrabutylammonium bromide.

10. Process according to claim 1, characterised in that diphenyl carbonate is prepared from phenol, CO and oxygen in the presence of sodium phenolate, of tetrabutylammonium bromide and of a catalyst.

## Revendications

1.  Procédé de préparation de carbonates de diaryle de formule

$$RO - CO - OR$$

par réaction d'un composé hydroxylé aromatique de formule

$$ROH,$$

formules dans lesquelles
R est un groupe aryle en $C_6$-$C_{12}$ substitué ou non substitué, de préférence un groupe phényle substitué ou non substitué, de préférence en particulier un groupe phényle non substitué,
avec CO et $O_2$ à une température de 30 à 200°C, de préférence de 30 à 150°C, en particulier de préférence de 40 à 120°C, et une pression de 1 à 150 bar, de préférence de 2 à 50 bar, en particulier de préférence de 5 à 25 bar, en présence d'un sel quaternaire, d'une base, d'un catalyseur métallique du groupe du platine et d'un cocatalyseur, caractérisé en ce qu'on travaille en outre en présence d'un promoteur hétérogène, qui représente un composé de formule

$$A_xB_yC_z$$

dans laquelle

A et B sont indépendamment l'un de l'autre un élément des groupes IIIA, IVA, VA, IIIB, IVB, VB, VIB ou VIIB du système périodique des éléments (classification de Mendelejew),
C est un élément de la deuxième période du système périodique des éléments (classification de Mendelejew),
x est un nombre de 1 à 3,
y est un nombre de 0 à 3 et
z est nombre de 1 à 12.

2. Procédé selon la revendication 1, caractérisé en ce qu'on utilise le promoteur hétérogène en une quantité de 0,001 à 50% en poids, de préférence de 0,01 à 10% en poids, de préférence en particulier de 0,1 à 5% en poids, par rapport à la quantité d'un composé hydroxylé aromatique, quand le promoteur est utilisé sous forme de suspension, ou on amène une charge de 0,1 à 20 g, de préférence de 0,2 à 10 g, de préférence en particulier de 0,2 à 5 g d'un composé hydroxylé aromatique par g de promoteur et par heure, quand le promoteur est placé dans un lit solide.

3. Procédé selon la revendication 1, caractérisé en ce que dans la formule $A_xB_yC_z$ pour le promoteur, C représente un élément du groupe du bore, du carbone, de l'azote et de l'oxygène.

4. Procédé selon la revendication 3, caractérisé en ce que le promoteur représente un oxyde, un carbure, un nitrure ou un borure métallique ou un matériau contenant du carbone.

5. Procédé selon la revendication 1, caractérisé en ce qu'on utilise pour sa réalisation, comme réacteur, un récipient agité ou une colonne à bulles ou une cascade de ces réacteurs, la cascade étant constituée de 2 à 15, de préférence de 2 à 10, de préférence en particulier de 2 à 5 réacteurs individuels.

6. Procédé selon la revendication 1, caractérisé en ce que le métal du groupe du platine est le palladium, de préférence sous forme de composés de palladium, de préférence en particulier sous forme de bromure de palladium ou d'acétylacétonate de palladium.

7. Procédé selon la revendication 1, caractérisé en ce qu'on introduit du phénol comme composé hydroxylé organique.

8. Procédé selon la revendication 1, caractérisé en ce qu'on utilise comme base une amine tertiaire, un phénolate alcalin ou un sel alcalin d'acides faibles, de préférence un carboxylate et/ou un phénolate alcalin, de préférence en particulier le phénolate de sodium.

9. Procédé selon la revendication 1, caractérisé en ce qu'on introduit comme sel quaternaire un sel de tétraalkylammonium ou de phosphonium, de préférence un sel de tétraalkylammonium, de préférence en particulier le bromure de tétrabutylammonium.

10. Procédé selon la revendication 1, caractérisé en ce qu'on prépare du carbonate de diphényle à partir de phénol, de CO et d'oxygène en présence de phénolate de sodium, de bromure de tétrabutylammonium et d'un catalyseur.